# EUROPEAN PATENT SPECIFICATION

(11) **EP 4 505 944 B1**
(45) Date of publication and mention of the grant of the patent: **25.03.2026**
(21) Application number: 24193304.3
(22) Date of filing: 07.08.2024
(51) Int. Cl.: A61B 6/08, A61B 6/00

(54) **METHOD AND DEVICE FOR DETERMINING TARGET DETECTION REGION OF IONIZATION CHAMBER, AND STORAGE MEDIUM**
VERFAHREN UND VORRICHTUNG ZUR BESTIMMUNG DES ZIELDETEKTIONSBEREICHS EINER IONISATIONSKAMMER UND SPEICHERMEDIUM
PROCÉDÉ ET DISPOSITIF DE DÉTERMINATION DE RÉGION DE DÉTECTION CIBLE DE CHAMBRE D'IONISATION, ET SUPPORT DE STOCKAGE

(30) Priority: 07.08.2023 CN 202310988873
(43) Date of publication of application: 12.02.2025
(73) Proprietor: Shanghai United Imaging Healthcare Co., Ltd., Shanghai 201807 (CN)
(72) Inventor: ZHANG, Bixiong, Shanghai, 201807 (CN)
(74) Representative: Murgitroyd & Company

(56) References cited:
- US-A1- 2005 169 425
- US-A1- 2011 249 791
- US-A1- 2023 157 660

## Description

### TECHNICAL FIELD

The present application relates to the technical field of medical equipment, and in particular to a method, and a device for determining a target detection region of an ionization chamber, and a storage medium.

### BACKGROUND

An Automatic Exposure Control (AEC) controls the exposure time of an X-ray machine and a total dose of X-rays by detecting doses of radiation passing through a to-be-scanned object or a target region via an X-ray sensor (an ionization chamber), thereby allowing images of different body parts or of different to-be-scanned objects obtained by X-ray imaging to have the same level of photosensitivity, thereby avoiding the questions of excessive dose differences between the images and poor image quality.

In the AEC, the dose of radiation passing through the target region of the scanned object is usually detected by using a gaseous/solid ionization chamber, and only the detection region of the ionization chamber can effectively detect the X-ray dose passing through the target region of the scanned object. Therefore, during a clinical usage, the detection region of the ionization chamber needs to be within the to-be-scanned object or the target region, otherwise the exposure dose may be relatively low, thus affecting the image quality. During clinical positioning, since there are no position identifications of the detection region of the ionization chamber on the surfaces of some movable objects, or the position identification of the detection region of the ionization chamber is blocked by a human body, an operator cannot obtain an accurate position of the detection region of the ionization chamber, and thus cannot accurately judge whether the detection region of the ionization chamber is within the corresponding to-be-scanned object or target region, and thus cannot reasonably adjust the detection region of the ionization chamber. Document US2023/157660A1 discloses systems and methods for performing an automated scan preparation for a scan of a target subject. Document US7120229B2 discloses an X-ray imaging control apparatus acquiring radiographic data obtained from an X-ray imaging apparatus which controls an X-ray dose upon X-ray imaging by detecting the X-ray dose in one or a plurality of detection regions, and displaying a radiographic image on the basis of the acquired radiographic data.

### SUMMARY

The present invention aims at overcoming the problem in the prior art that an operator cannot reasonably adjust a detection region of an ionization chamber for the reason that no position identification of a detection region of an ionization chamber is provided or a position identification is blocked by a human body. A method and a device for determining a target detection region of an ionization chamber, and a storage medium are provided.

The present application solves the above-mentioned technical problem through the method defined in claim 1.

In some embodiments, identifying the target region based on the image includes: identifying a plurality of human body feature points based on the image, and obtaining second positions of the plurality of human body feature points in the spatial coordinate system; determining the target region according to the plurality of human body feature points and the second positions.

In some embodiments, determining the target region according to the plurality of human body feature points and the second positions includes: determining target human body feature points corresponding to a target human-body part according to the plurality of human body feature points; taking a center of the second positions corresponding to the target human feature points as a center point, and determining a region within a preset distance from the center point as the target region.

In some embodiments, the method further includes: controlling a display screen to display the target region and the target detection region.

In some embodiments, the target detection region is moved on the display screen to make the first position of the ionization chamber coincide with the target position.

In some embodiments, the method further includes: outputting the plurality of human body feature points and the target detection region; taking a human body feature point selected by a user as a target feature point; recalculating the target region and redetermining the target detection region according to the position of the target feature point, and outputting the target feature point and the redetermined target detection region.

In some embodiments, the method further includes: controlling a projection device to project at least one of a center identification of the target detection region and a boundary identification of the target detection region onto a surface of the to-be-scanned object.

In some embodiments, the ionization chamber includes one or more dose receiving units. The selectable detection region of the ionization chamber includes all of the one or more dose receiving units of the ionization chamber. The target detection region of the ionization chamber includes only dose receiving units in a working state.

In some embodiments, the ionization chamber is a dose-receiving-unit array consisting of multiple dose receiving units.

In some embodiments, identifying the plurality of human body feature points based on the image, and obtaining the second positions of the plurality of human body feature points in the spatial coordinate system, include: identifying the plurality of human body feature points in an image; constructing a human body model in the spatial coordinate system; and determining positions of the plurality of human body feature points in a three-dimensional spatial coordinate system based on positions of the plurality of human body feature points in the human body model, wherein the positions of the plurality of human body feature points in the three-dimensional spatial coordinate system are the second positions.

In some embodiments, identifying the plurality of human body feature points in the image, includes: inputting sample images marked with human body feature points into a neural network model to train a recognition model for the human body feature points; inputting the image including the target region of the to-be-scanned object acquired by the image collector into the trained recognition model to identify the plurality of human body feature points.

The present application also provides a device as defined in claim 12.

The image acquiring module is configured to obtain an image including a target region of the to-be-scanned object acquired by the image collector.

The image recognizing module is configured to identify the target region based on the image.

The first position obtaining module is configured to obtain a first position of the ionization chamber in a spatial coordinate system.

The selectable detection region calculating module is configured to obtain a selectable detection region of the ionization chamber based on the first position.

The target detection region determining module is configured to, when the target region is outside the selectable detection region, obtain a target position of the ionization chamber, and control a driving device of the ionization chamber to move the ionization chamber to enable the target position to coincide with the first position, where when the ionization chamber is located at the target position, the selectable detection region of the ionization chamber at least partially overlaps the target region; and when the target region is within the selectable detection region, select and determine the target detection region from the selectable detection region, where a minimum distance between a boundary of the target detection region and a boundary of the target region is within a preset boundary distance, and the target detection region of the ionization chamber is within the target region.

The present application also provides an electronic device, including a memory and a processor. The memory stores a computer program executable on the processor. The processor, when executing the computer program, performs any one of the methods for determining the target detection region of the ionization chamber above.

The present application also provides a non-transitory computer-readable storage medium, having a computer program stored thereon. The computer program, when being executed by a processor, performs any one of the methods for determining the target detection region of the ionization chamber above.

The present application also provides a program product, including program codes. When the program product is run on a terminal, the program codes cause the terminal to perform any one of the methods for determining the target detection region of the ionization chamber above.

The beneficial effects of the present application are as follows. The method for determining the target detection region of the ionization chamber includes obtaining an image including a target region of a to-be-scanned object acquired by an image collector; identifying the target region based on the image; obtaining a first position of the ionization chamber in a spatial coordinate system; obtaining a selectable detection region of the ionization chamber based on the first position; and determining the target detection region of the ionization chamber according to the target region and the selectable detection region so that the target detection region of the ionization chamber is within the target region, thereby realizing the determination of the target detection region of the ionization chamber without visually inspecting, by the operator, the position of the detection region of the ionization chamber, thereby improving the determination efficiency and the determination accuracy, achieving a better imaging position, ensuring the quality of images, and enhancing the user's experience.

### BRIEF DESCRIPTION OF THE DRAWINGS

FIG. 1 is a flow chart of a method for determining a target detection region of an ionization chamber according to an embodiment of the present application.
FIG. 2 is a view showing an application scenario of the method for determining the target detection region of the ionization chamber according to some embodiments of the present application.
FIG. 3 is a view showing a structure of an example of a conventional ionization chamber in the related art.
FIG. 4 is a view showing a structure of an adjustable ionization chamber according to an embodiment of the method for determining the target detection region of the ionization chamber of the present application.
FIG. 5 shows an example of a human body model in a target detection region of an ionization chamber according to an embodiment of the present application.
FIG. 6 shows an example of a human body model in a target detection region of an ionization chamber while an object's chest is photographed according to an embodiment of the present application.
FIG. 7 is a view showing an ionization chamber receiving X-rays in a target detection region of an ionization chamber while an object's chest is photographed according to an embodiment of the present application.
FIG. 8 is a flow chart of a step S12 of the method for determining the target detection region of the ionization chamber according to an embodiment of the present application.
FIG. 9 is a flow chart of a step S122 of the method for determining the target detection region of the ionization chamber according to an embodiment of the present application.
FIG. 10 is a flow chart of a step S15 of the method for determining the target detection region of the ionization chamber according to an embodiment of the present application.
FIG. 11 shows an example of a target region outside a selectable detection region according to an embodiment of the method for determining the target detection region of the ionization chamber of the present application.
FIG. 12 shows an example of a target region overlapping a selectable detection region according to an embodiment of the method for determining the target detection region of the ionization chamber of the present application.
FIG. 13 is a flow chart of the method for determining the target detection region of the ionization chamber according to another embodiment of the present application.
FIG. 14 is a view showing circuitry modules of a device for determining a target detection region of an ionization chamber according to an embodiment of the present application.
FIG. 15 is a view showing a structure of an electronic device according to an embodiment of the present application.

### DETAILED DESCRIPTION OF THE EMBODIMENTS

The present application is further described hereinafter through embodiments, but is not limited to the scope of the embodiments.

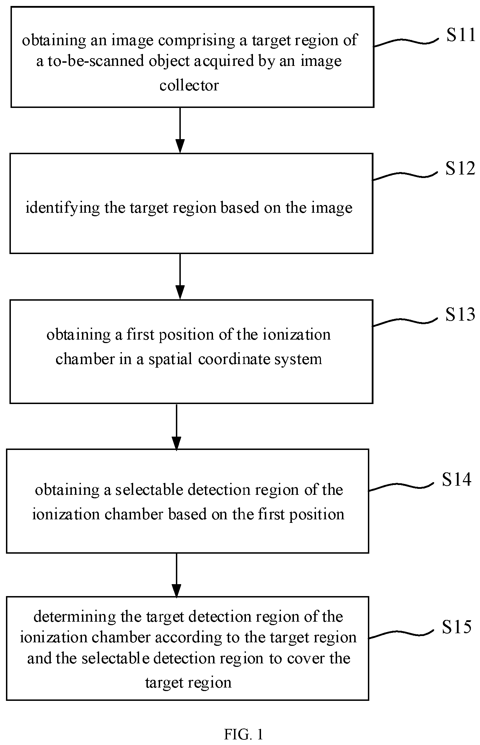

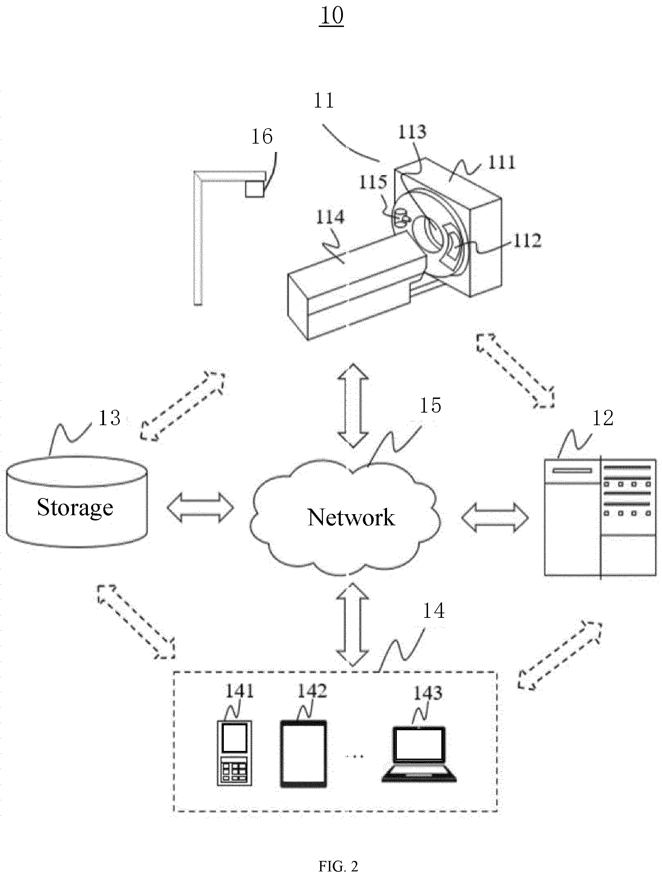

Referring to FIG.1, an embodiment of the present application provides a method for determining a target detection region of an ionization chamber. The method for determining the target detection region of the ionization chamber of the embodiment of the present application is applied to a scenario of FIG. 2. As shown in FIG.2, a system 10 for determining a target detection region of an ionization chamber may include a scanning device 11, a processor 12, a memory 13, one or more terminals 14, a network 15 and a projection device 16. The scanning device 11 may include a gantry 111, a detector 112, a detection region 113, a scanning table 114, a radiation source 115, and an ionization chamber 116. The scanning device 11, the processor 12, the memory 13, the terminal 14 and/or the projection device 16 may be interconnected and/or communicated by wireless connection, wired connection or a combination thereof. The connection between the components of the system 10 may be variable. As shown in FIG.1, the method for determining the target detection region of the ionization chamber of the present application includes steps S11 to S15.

In step S11, an image including a target region of a to-be-scanned object acquired by an image collector is obtained.

In step S12, the target region is identified based on the image.

In step S13, a first position of the ionization chamber is obtained. Specifically, the first position of the ionization chamber is a position of the ionization chamber in a spatial coordinate system.

In step S14, a selectable detection region of the ionization chamber is obtained based on the first position.

In step S15, a target detection region of the ionization chamber is determined according to the target region and the selectable detection region so that the target detection region of the ionization chamber is within the target region.

The target region of the to-be-scanned object is a region of the to-be-scanned object that needs to be photographed and examined, and may be a human-body part or partial human-body part, such as an ankle, a finger joint, a thoracic vertebra, a knee, etc. In an embodiment, for a target region, there may be multiple target detection regions. The image collector may be a detector 112 installed or integrated in the scanning device 11, or the image collector may be an optical image acquisition device, such as a camera installed or integrated in the scanning device 11, and the type and the number of the image collectors are not limited herein. For example, two two-dimensional cameras or one three-dimensional camera may be used to collect images.

The ionization chamber is generally arranged between the to-be-scanned object and a portion of the scanning device 11, specifically, between the to-be-scanned object and the detector 112. The ionization chamber is a separate component on a digital radiography (DR) device, and is used to receive doses of radiation (such as X-ray), and after the dose of radiation reaches a certain threshold, a signal is fed back to the radiation source to force the radiation source to stop radiating radiation. Ionization chambers generally include traditional ionization chambers and adjustable ionization chambers.

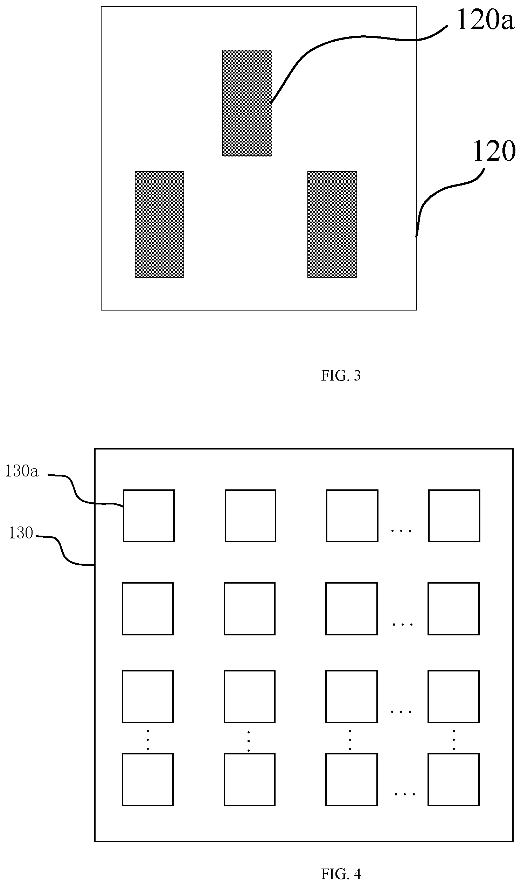

FIG. 3 is a view showing a structure of an example of a conventional ionization chamber in the related art. As can be seen from the figure, the ionization chamber 120 generally includes one or more dose receiving units 120a. The dose receiving unit 120a is configured to receive the doses of X-rays, and the signal is fed back when the dose of the received X-rays reaches the threshold. FIG. 3 shows an ionization chamber 120 including three dose receiving units 120a. Of course, in other embodiments, the ionization chamber 120 may also include over three dose receiving units, or may be a dose-receiving-unit array consisting of multiple dose receiving units. The conventional ionization chamber is coupled to a detector and unmovable, and has an un-adjustable size and an un-adjustable range.

The ionization chamber used in the present application is an ionization chamber improved based on the traditional ionization chamber, and has a moving unit. The moving unit includes a driving device configured to determine the position of the ionization chamber, and a determination unit configured to determine the range and size of the ionization chamber, for example, by providing a control switch for each dose receiving unit to control the dose receiving unit to be in a working state. At least the position and size of the ionization chamber used in the present application are adjustable, the selectable detection region of the ionization chamber includes all dose receiving units of the ionization chamber, and the target detection region of the ionization chamber includes only the dose receiving units in a working state. The working state means that the target detection region, after activated, receives the dose of X-ray.

FIG. 4 is a view showing a structure of an exemplary adjustable ionization chamber, and the adjustable ionization chamber 130 is integrated in the detector with a function of the AEC. For example, the adjustable ionization chamber 130 is a 40 × 40 dose-receiving-unit array, and any one of or any combination of 1600 dose receiving units 130a may be activated individually to detect the doses of X-rays. The selectable detection region includes all 1600 dose receiving units 130a, and the target detection region includes only the activated dose receiving units.

The ionization chamber to which the method for determining the target detection region of the ionization chamber of this embodiment is applied may be a modified traditional ionization chamber or an adjustable full-field ionization chamber. The first position of the ionization chamber may be obtained by a position sensor or by a motion encoder. Since the installation position, imaging angle and imaging range of the image collector are controllable and may be determined, the position of the object (including the target region of the to-be-scanned object) in the image acquired by the image collector may be identified from the image. The target region identified based on the image and the ionization chamber are placed in the same spatial coordinate system, and the spatial position relationship between the target region and the ionization chamber is obtained based on the positions of the target region and of the ionization chamber in the spatial coordinate system, thus obtaining the spatial position relationship between the target region and the selectable detection region of the ionization chamber, for example, coinciding with each there or overlapping each other, etc. Base on this spatial position relationship, the target detection region of the ionization chamber may be determined conveniently and visually, which may aid an operator in allowing the target detection region of the ionization chamber to be in a suitable position and to have a suitable size and shape in a process of positioning the to-be-scanned object, so that a better positioning effect is achieved, and that the AEC of the target region is more accurate, thereby more accurately controlling the exposure time of the X-ray machine and the total dose of the X-ray, obtaining images with good exposure quality, and ensuring the quality of photographed images.

A human body model may be constructed, and the spatial positional relationship between the target region and the selectable detection region of the ionization chamber may be obtained through the positional relationships between human body feature points and the human body model and the ionization chamber in the same spatial coordinate system.

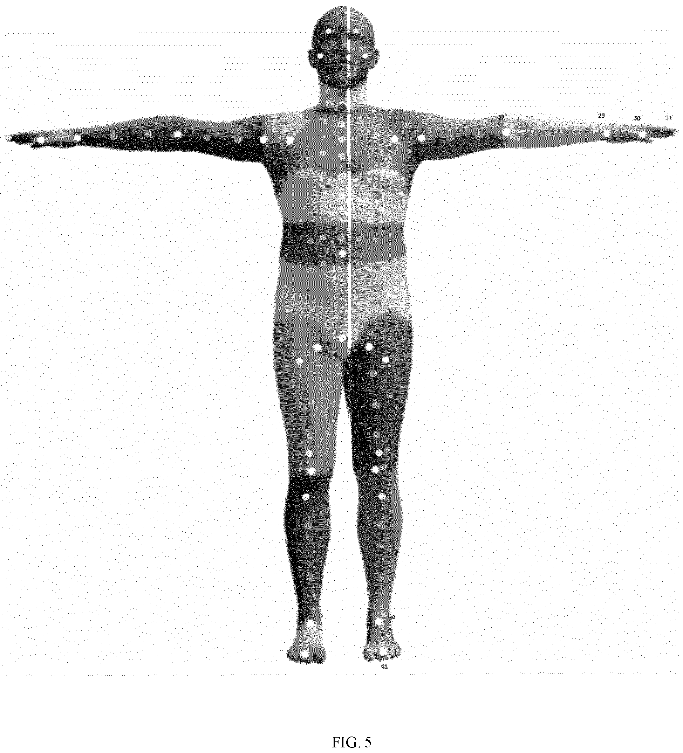

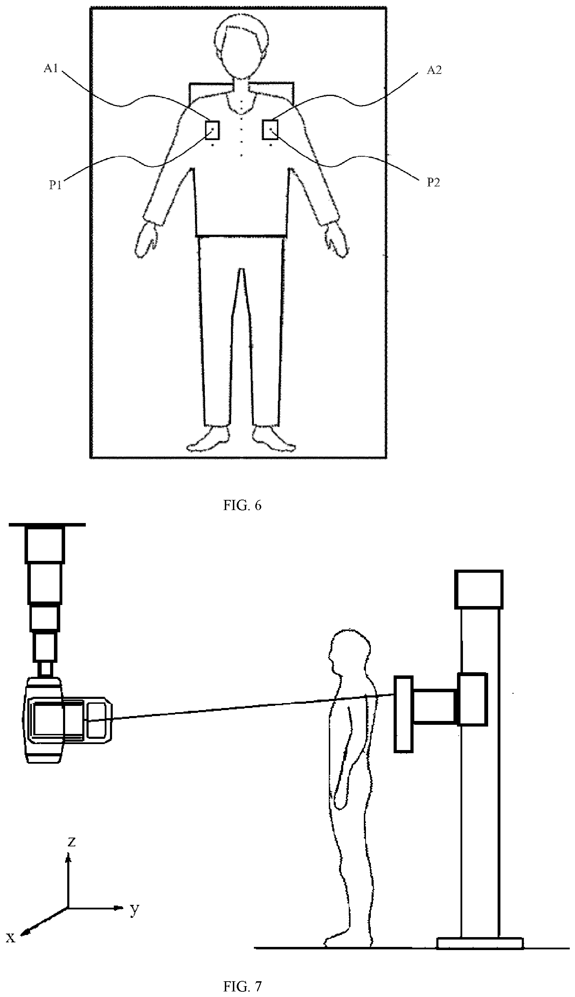

FIG. 5 shows an example of a human body model, and dots in the figure represent human body feature points. FIG. 6 shows an example of a human body model while an object's chest is photographed, and dots in the figure represent the human body feature points. FIG. 7 is a view showing an ionization chamber receiving X-rays while an object's chest is photographed, and a straight line in the figure passing through the human body represents the X-ray. In order to achieve a good imaging effect, the spatial positional relationship between the target region and the selectable detection region of the ionization chamber may be obtained through the positional relationships between the human body feature points and the human body model and the ionization chamber.

In this embodiment, the human body feature points are identified based on the images, the human model in the spatial coordinate system is constructed, and the target region of the to-be-scanned object is determined. Then the spatial position relationship between the target region and the selectable detection region of the ionization chamber is obtained based on the first position of the ionization chamber in the same spatial coordinate system. The target detection region of the ionization chamber is determined based on the spatial position relationship, thus realizing the determination of the target detection region of the ionization chamber without visually inspecting, by the operator, the position of the detection region of the ionization chamber, thereby improving the determination efficiency and the determination accuracy, achieving a better imaging position, ensuring the quality of images, and enhancing the user's experience.

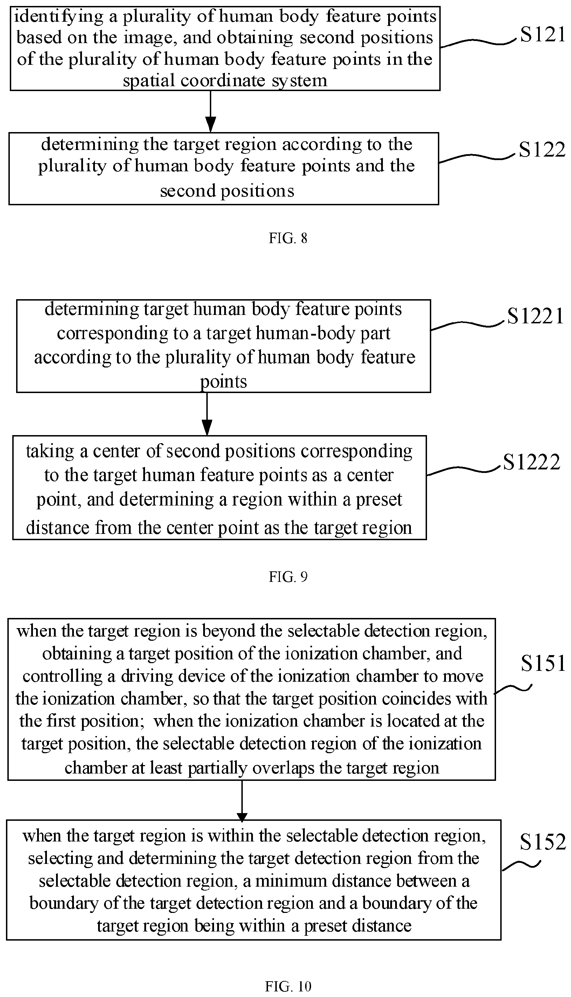

In an embodiment of the present application, referring to FIG. 8, step S12 of identifying the target region based on the image includes steps S121 and S122.

In step S121, a plurality of human body feature points are identified based on the image, and second positions of the human body feature points in the spatial coordinate system are obtained.

In step S122, the target region is determined according to the human body feature points and the second positions.

The human body feature points of the to-be-scanned object are identified based on the image, and the second positions of the human body feature points are obtained. That is, the human body feature points are identified in the image, and then the human body model is constructed in the spatial coordinate system. Based on the positions of the human body feature points in the human body model, the positions of the human body feature points in the three-dimensional spatial coordinate system, namely the second positions, are determined, so that the human body feature points are also positioned in the same spatial coordinate system mentioned above, and the target region of the to-be-scanned object may be determined based on the identified human body feature points. The image may be a two-dimensional or three-dimensional image.

Specifically, the human body feature points in the image may be identified by a neural network algorithm. Firstly, sample images marked with human body feature points are inputted into a neural network model to train a recognition model for human body feature points. Then, the image including the target region of the to-be-scanned object acquired by the image collector is inputted into the trained recognition model to identify the human body feature points.

In an embodiment of the present application, a specific implementation method for identifying the target region based on the image is provided, which is helpful in automatically determining the target detection region of the ionization chamber.

In an embodiment of the present application, referring to FIG. 9, the step S122 of determining the target region according to the human body feature points and the second positions includes steps S1221 and S1222.

In step S1221, target human body feature points corresponding to a target human-body part are determined according to the human body feature points.

In step S1222, a center of the second positions corresponding to the target human feature points is taken as a center point, and a region within a preset distance from the center point is determined as the target region.

This embodiment provides a specific implementation method of identifying the plurality of human body feature points based on the image and determining the target region based on the human body feature points and the second positions, and defines an implementation method of determining the target human body feature points corresponding to the target human-body part based on the human body feature points and determining the target region based on the center point of the target human body feature points. The target human-body part refers to a part of the to-be-scanned object, such as an arm, a chest, and an antisternum, etc. By taking the center of the second positions corresponding to the target human feature points as a center point, and determining the region within the preset distance from the center point as the target region, the determined target region may be more accurate. After the target detection region is determined according to the target region, images of different body parts or of different to-be-scanned objects obtained by X-ray imaging have high resolutions and high signal-to-noise ratios.

In an embodiment of the present application, referring to FIG. 10, step S15 of determining the target detection region of the ionization chamber according to the target region and the selectable detection region so that the target detection region of the ionization chamber is within the target region, includes steps S151 and S152.

In Step S151, when the target region is outside the selectable detection region, the target position of the ionization chamber is obtained, and the driving device of the ionization chamber is controlled to move the ionization chamber so that the target position coincides with the first position. When the ionization chamber is located at the target position, the selectable detection region of the ionization chamber at least partially overlaps the target region. In an embodiment, when the target region is outside the selectable detection region, the target position of the ionization chamber is obtained, and a driving device of the object is controlled to move the object so that the selectable detection region of the ionization chamber at least partially overlaps the target region. Alternatively, in an embodiment, when the target position coincides with the first position of the ionization chamber, one or more of the selectable detection regions of the ionization chamber may be adjusted so that the one or more selectable detection regions of the ionization chamber at least partially overlap the target region to obtain the target detection region.

In Step S152, when the target region is within the selectable detection region, the target detection region is selected and determined from the selectable detection region, where a minimum distance between a boundary of the target detection region and a boundary of the target region is within a preset boundary distance.

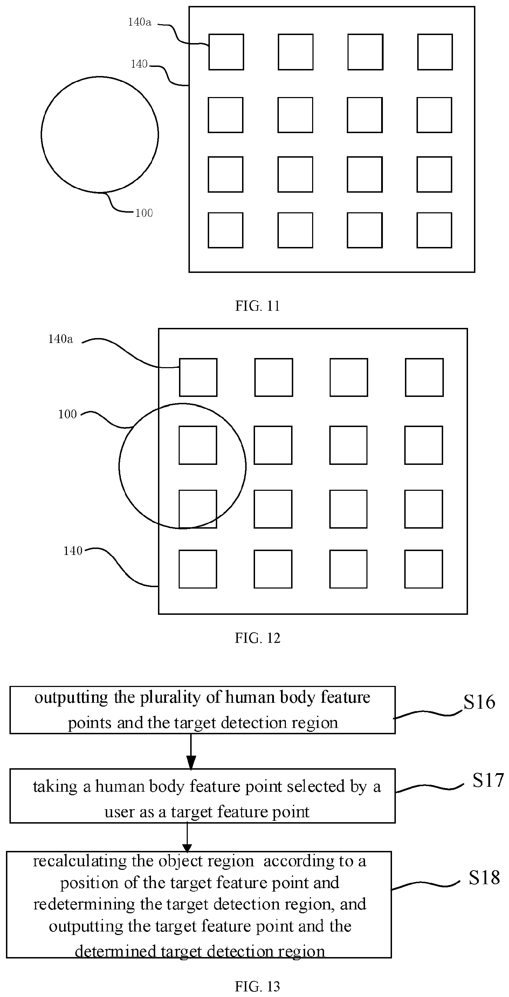

The target region is outside the selectable detection region, which means that the target region is not within the detection range of the ionization chamber. FIG. 11 shows an example of a target region outside a selectable detection region. In the figure, the ionization chamber 140 includes a plurality of dose receiving units 140a, and the selectable detection region includes all of the dose receiving units 140a, and the target region 100 is outside the detection range of the ionization chamber 140.

When the target region is outside the selectable detection region, the ionization chamber is moved from the first position, namely, the starting point, to the target position, namely, the end point. Since the first position and the target position are in the same coordinate system, it is convenient to calculate the moving direction and the moving distance of the ionization chamber. According to the structure of the driving device, the moving direction and the moving distance are decomposed into moving vectors executable by the driving device.

The driving device may include guide rails arranged along the horizontal axis and the vertical axis of the ionization chamber, respectively, and each guide rail is provided with a driving motor and a hinge. The driving motor drives the hinge to drive the ionization chamber to move along the guide rail based on the moving vector until the first position coincides with the target position. The selectable detection region of the moved ionization chamber at least partially overlaps the target region. As shown in FIG. 12, the ionization chamber 140 includes a plurality of dose receiving units 140a, and under the drive of the driving device, the selectable detection region of the moved ionization chamber 140 at least partially overlaps the target region 100 of the to-be-scanned object. This embodiment is an example of the structure of the driving device, and the driving device may also have any other structure.

The above-mentioned related operations of moving the ionization chamber are applicable to the improved traditional ionization chamber and the adjustable ionization chamber. In addition, for the adjustable ionization chamber and the improved traditional ionization chamber, the target detection region may be determined by controlling the corresponding dose receiving units to be in a working state.

The embodiment of the present application provides a specific implementation method for determining the target detection region of the ionization chamber to achieve a good imaging effect, thereby further improving the determination efficiency, improving the determination accuracy, achieving a better positioning and imaging effect, ensuring the image quality, and enhancing the user's experience.

In an embodiment of the present application, the determination method further includes: controlling a display screen to display the target region and the target detection region.

The method for determining the target detection region of the ionization chamber of the present application is applied to the system 10 for determining the target detection region of the ionization chamber shown in FIG. 2. The terminal 14 in the system 10 may include a mobile device 141, a tablet computer 142, a laptop computer 143, etc. The processor 12 may send a control instruction to the terminal 14, so that the target region and the target detection region are displayed on the display screen of the terminal 14. In this embodiment, the target region and the target detection region are displayed on the display screen, thus making it convenient for the operator to visually get the spatial position relationship between the target region and the target detection region, and further providing convenience for determining the target detection region of the ionization chamber.

In an embodiment, the target detection region is moved on the display screen to make the first position of the ionization chamber coincide with the target position.

The target detection region may be moved on the display screen by inputting coordinates of the target position, dragging the mouse, and touch control, etc.

The size of the target detection region may be determined on the display screen by inputting the size of the target detection region, dragging the boundary of the region with the mouse, adjusting the boundary of the region through touch control, etc., so as to cover a relatively small part of the to-be-scanned object in photographing and achieve a better effect of the AEC.

The shape of the target detection region may also be determined on the display. A default shape of the ionization chamber is rectangular or circular, and the shape may be determined based on the default shape.

In this embodiment, the target detection region of the ionization chamber may be determined through various operations on the display screen to achieve a better imaging effect, thus further improving the determination efficiency, improving the determination accuracy, achieving a better positioning and photographing effect, ensuring the image quality, and improving the user's experience.

In an embodiment of the present application, referring to FIG. 13, the method for determining the target detection region of the ionization chamber further includes steps S16 to S18.

In Step S16, the human body feature points and the target detection region are outputted.

In Step S17, a human body feature point selected by a user is taken as a target feature point.

In Step S18, the target region is recalculated according to the position of the target feature point and the target detection region is redetermined, and the target feature point and the redetermined target detection region are outputted. In an embodiment, the target region is recalculated by taking the position of the target feature point as a center and determined by a region within a preset distance from the center point.

The human body feature points and the target detection region may be outputted through the display screen in the form of text data and images.

Referring to FIGS. 6 and 7, when the image collector intelligently identifies a human-body part, the positions P1 and P2 of human body feature points (such as joint points) in the photographed human-body part and the target detection regions A1 and A2 of the ionization chamber may be intelligently highlighted on the human body model, and the non-selected areas are grayed. When the user expects to change the position of the automatic exposure region, a human body feature point in the target part may be activated as a target feature point, and the selection is taken into effect. The system re-executes step S12 (including steps S121 and S122) to step S15 according to the feature point expected and selected by the user, namely, the target feature point. That is, the coordinates of the position of the target feature point are re-projected, and the target region is obtained based on the target feature point, and a new position (a new first position) of the ionization chamber is calculated, and the selectable detection region of the ionization chamber is obtained according to the new first position. The target detection region of the ionization chamber is determined according to the target region and the selectable detection region so that the target detection region of the ionization chamber is within the target region, thereby realizing a manual selection and determination of the target detection region of the ionization chamber on the interface based on the human body feature points.

In an embodiment of the present application, the method for determining the target detection region of the ionization chamber further includes: controlling a projection device to project a center identification of the target detection region and/or a boundary identification of the target detection region onto the surface of the to-be-scanned object.

In this embodiment, for example, before scanning, the processor 12 may send the corresponding identifications of the target detection region of the ionization chamber to the projection device 16, and control the projection device 16 to project the corresponding identifications of the target detection region on the surface of the to-be-scanned object, so that the operator may visually get the spatial position relationship between the target region and the target detection region, thus further making it convenient to determine the target detection region of the ionization chamber.

An embodiment of the present invention further provides an X-ray imaging device having the function of adjusting a target detection region of an ionization chamber. The X-ray imaging device includes an image collector, the ionization chamber, a detector, and an X-ray source. The detector is configured to obtain an X-ray image of a scanned object including a target area. The ionization chamber is arranged on the detector, and the target detection region of the ionization chamber is adjusted according to the target area. According to exposure signals obtained in the target detection area of the ionization chamber during an exposure process, the X-ray source is automatically controlled to stop the exposure to obtain the X-ray image.

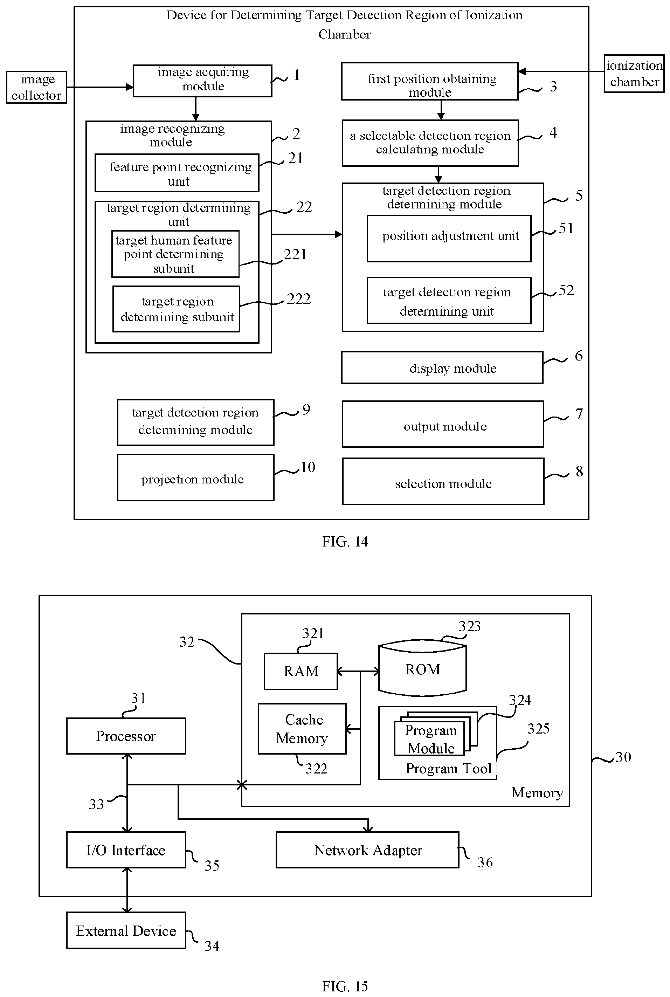

The present application also provides a device for determining a target detection region of an ionization chamber. Referring to FIG. 14, the device includes an image acquiring module 1, an image recognizing module 2, a first position obtaining module 3, a selectable detection region calculating module 4, and a target detection region determining module 5.

In an embodiment, the device for determining the target detection region of the ionization chamber may be applied to the scenario of FIG. 2. As shown in FIG.2, the system for determining the target detection region of the ionization chamber includes a scanning device, and the scanning device includes the ionization chamber. The ionization chamber is arranged between a to-be-scanned object and the scanning device, and the scanning device is provided with an image collector. As shown in FIG. 14, in the device for determining the target detection region of the ionization chamber, an input of the image acquiring module 1 is connected to an image collector, an output of the image acquiring module 1 is connected to an input of the image recognizing module 2. An input of the first position obtaining module 3 is connected to the ionization chamber, and an output of the first position obtaining module 3 is connected to an input of the selectable detection region calculating module 4. An input of the target detection region determining module 5 is connected to an output of the selectable detection region calculating module 4, and another input of the target detection region determining module 5 is connected to an output of the image recognizing module 2. Arrows in the figure denote the directions of signals.

The image acquiring module 1 is configured to obtain an image including a target region of a to-be-scanned object acquired by an image collector.

The image recognizing module 2 is configured to identify a target region based on the image.

The first position obtaining module 3 is configured to obtain a first position of the ionization chamber in a spatial coordinate system.

The selectable detection region calculating module 4 is configured to obtain a selectable detection region of the ionization chamber based on the first position.

The target detection region determining module 5 is configured to determine a target detection region of the ionization chamber according to the target region and the selectable detection region so that the target detection region of the ionization chamber is within the target region..

The target region of the to-be-scanned object is a region of the to-be-scanned object that needs to be photographed and examined, and may be a human-body part or partial human-body part, such as an ankle, a finger joint, a thoracic vertebra, a knee, etc. The image collector may be a detector 112 installed or integrated in the scanning device 11, or the image collector may be a camera installed or integrated in the scanning device 11, and the type and the number of the image collectors are not limited herein. For example, two two-dimensional cameras or one three-dimensional camera may be used to collect images.

The ionization chamber is generally arranged between the to-be-scanned object and the scanning device 11, specifically, between the to-be-scanned object and the detector 112. The ionization chamber is a separate component on a digital radiography (DR) device, and is used to receive doses of radiation (such as X-ray), and after the dose of radiation reaches a certain threshold, a signal is fed back to the radiation source to force the radiation source to stop radiating radiation. Ionization chambers generally include traditional ionization chambers and adjustable ionization chambers.

FIG. 3 is a view showing a structure of an example of a conventional ionization chamber in the related art. As can be seen from the figure, the ionization chamber 120 generally includes one or more dose receiving units 120a. The dose receiving unit 120a is configured to receive the doses of X-rays, and a signal is fed back when the dose of the received X-rays reaches the threshold. FIG. 3 shows an ionization chamber 120 including three dose receiving units 120a. Of course, in other embodiments, the ionization chamber 120 may also include over three dose receiving units, or may be a dose-receiving-unit array consisting of multiple dose receiving units. The conventional ionization chamber is coupled to a detector and unmovable, and has an un-adjustable size and an un-adjustable range.

The ionization chamber used in the present application is an ionization chamber improved based on the traditional ionization chamber, and has a moving unit. The moving unit includes a driving device configured to determine the position of the ionization chamber, and a determination unit configured to determine the range and size of the ionization chamber, for example, by providing a control switch for each dose receiving unit to control the dose receiving unit to be in a working state. At least the position and size of the ionization chamber used in the present application are adjustable, the selectable detection region of the ionization chamber includes all dose receiving units of the ionization chamber, and the target detection region of the ionization chamber includes only the dose receiving units in a working state.

FIG. 4 is a view showing a structure of an exemplary adjustable ionization chamber, and the adjustable ionization chamber 130 is integrated in the detector with a function of the AEC. For example, the adjustable ionization chamber 130 is a 40 × 40 dose-receiving-unit array, and any one of or any combination of 1600 dose receiving units 130a may be activated individually to detect the doses of X-rays. The selectable detection region includes all 1600 dose receiving units 130a, and the target detection region includes only the activated dose receiving units.

The ionization chamber to which the method for determining the target detection region of the ionization chamber of this embodiment is applied may be a modified traditional ionization chamber or an adjustable full-field ionization chamber. The first position of the ionization chamber may be obtained by a position sensor or by a motion encoder. Since the installation position, imaging angle and imaging range of the image collector are controllable and may be determined, the position of the object (including the target region of the to-be-scanned object) in the image acquired by the image collector may be identified from the image. The target region identified based on the image and the ionization chamber are placed in the same spatial coordinate system, and the spatial position relationship between the target region and the ionization chamber is obtained based on the positions of the target region and the ionization chamber in the spatial coordinate system, thus obtaining the spatial position relationship between the target region and the selectable detection region of the ionization chamber, for example, coinciding with or overlapping each other, etc. Base on this spatial position relationship, the target detection region of the ionization chamber may be determined conveniently and visually, which may aid an operator in allowing the target detection region of the ionization chamber to be in a suitable position and to have a suitable size and shape in a process of positioning the to-be-scanned object, so that a better positioning effect is achieved, and that the AEC of the target region is more accurate, thereby more accurately controlling the exposure time of the X-ray machine and the total dose of the X-ray, obtaining images with good exposure quality, and ensuring the quality of photographed images.

A human body model may be constructed, and the spatial positional relationship between the target region and the selectable detection region of the ionization chamber may be obtained through the positional relationships between human body feature points and the human body model and the ionization chamber in the same spatial coordinate system.

FIG. 5 shows an example of a human body model, and dots in the figure represent the human body feature points. FIG. 6 shows an example of a human body model while an object's chest is photographed, and dots in the figure represent the human body feature points. FIG. 7 is a view showing an ionization chamber receiving X-rays while an object's chest is photographed, and a straight line in the figure passing through the human body represents the X-ray. In order to achieve a good imaging effect, the spatial positional relationship between the target region and the selectable detection region of the ionization chamber may be obtained through the positional relationships between the human body feature points and the human body model and the ionization chamber.

In this embodiment, the human body feature points are identified based on the images, the human model in the spatial coordinate system is constructed, and the target region of the to-be-scanned object is determined. Then the spatial position relationship between the target region and the selectable detection region of the ionization chamber is obtained based on the first position of the ionization chamber in the same spatial coordinate system. The target detection region of the ionization chamber is determined based on the spatial position relationship, thus realizing the determination of the target detection region of the ionization chamber without visually inspecting, by the operator, the position of the detection region of the ionization chamber, thereby improving the determination efficiency and the determination accuracy, achieving a better imaging position, ensuring the quality of images, and enhancing the user's experience.

In an embodiment of the present application, the image recognizing module 2 includes: a feature point recognizing unit 21 and a target region determining unit 22.

The feature point recognizing unit 21 is configured to identify a plurality of human body feature points based on the image, and obtain second positions of the human body feature points in the spatial coordinate system.

The target region determining unit 22 is configured to determine the target region according to the human body feature points and the second positions.

The human body feature points of the to-be-scanned object are identified based on the image, and the second positions of the human body feature points are obtained. That is, the human body feature points are identified in the image, and then the human body model is constructed in the spatial coordinate system. Based on the positions of the human body feature points in the human body model, the positions of the human body feature points in the three-dimensional spatial coordinate system, namely the second positions, are determined, so that the human body feature points are also positioned in the same spatial coordinate system mentioned above, and the target region of the to-be-scanned object may be determined based on the identified human body feature points.

Specifically, the human body feature points in the image may be identified by a neural network algorithm. Firstly, sample images marked with human body feature points are inputted into a neural network model to train a recognition model for human body feature points. Then, the image including the target region of the to-be-scanned object acquired by the image collector is inputted into the trained recognition model to obtain identified human body feature points.

In an embodiment of the present application, a specific implementation method for identifying the target region based on the image is provided, which is helpful in automatically determining the target detection region of the ionization chamber.

In an embodiment of the present application, the target region determining unit 22 includes: a target human feature point determining subunit 221 and a target region determining subunit 222.

The target human body feature point determination subunit 221 is configured to determine target human body feature points corresponding to a target human-body part according to the human body feature points.

The target region determination subunit 222 is configured to take a center of the second positions corresponding to the target human feature point as a center point, and determine a region within a preset distance from the center point as the target region.

This embodiment provides the specific implementation method of identifying the plurality of human body feature points based on the image and determining the target region based on the human body feature points and the second positions, and defines an implementation method of determining the target human body feature points corresponding to the target human-body part based on the human body feature points and determining the target region based on the center point of the target human body feature points. The target human-body part refers to a part of the to-be-scanned object, such as an arm, etc.

In an embodiment of the present application, the target detection region determining module 5 includes: a position adjustment unit 51 and a target detection region determining unit 52.

The position adjustment unit 51 is configured to, when the target region is outside the selectable detection region, obtain the target position of the ionization chamber, and control the driving device of the ionization chamber to move the ionization chamber so that the first position and the target position coincide with each other. When the ionization chamber is located at a target position, the selectable detection region of the ionization chamber at least partially overlaps the target region.

The target detection region determining unit 52 is configured to, when the target region is within the selectable detection region, select and determine the target detection region from the selectable detection region, where a minimum distance between a boundary of the target detection region and a boundary of the target region is within a preset boundary distance.

The target region is outside the selectable detection region, which means that the target region is not within the detection range of the ionization chamber. FIG. 11 shows an example of a target region outside a selectable detection region. In the figure, the ionization chamber 140 includes a plurality of dose receiving units 140a, and the selectable detection region includes all of the dose receiving units 140a, and the target region 100 is outside the detection range of the ionization chamber 140.

When the target region is outside the selectable detection region, the ionization chamber is moved from the first position, namely, the starting point, to the target position, namely, the end point. Since the first position and the target position are in the same coordinate system, it is convenient to calculate the moving direction and the moving distance of the ionization chamber. According to the structure of the driving device, the moving direction and the moving distance are decomposed into moving vectors executable by the driving device.

The driving device may include guide rails arranged along the horizontal axis and the vertical axis of the ionization chamber, respectively, and each guide rail is provided with a driving motor and a hinge. The driving motor drives the hinge to drive the ionization chamber to move along the guide rail based on the moving vector until the first position coincides with the target position. The selectable detection region of the moved ionization chamber at least partially overlaps the target region. As shown in FIG. 12, the ionization chamber 140 includes a plurality of dose receiving units 140a, and under the drive of the driving device, the selectable detection region of the moved ionization chamber 140 at least partially overlaps the target region 100 of the to-be-scanned object. This embodiment is an example of the structure of the driving device, and the driving device may also have any other structure.

The above-mentioned related operations of moving the ionization chamber are applicable to the improved traditional ionization chamber and the adjustable ionization chamber. In addition, for the adjustable ionization chamber and the improved traditional ionization chamber, the target detection region may be determined by controlling the corresponding dose receiving units to be in a working state.

The embodiment of the present application provides a specific implementation method for determining the target detection region of the ionization chamber to achieve a good imaging effect, thereby further improving the determination efficiency, improving the determination accuracy, achieving a better positioning and imaging effect, ensuring the image quality, and enhancing the user's experience.

In an embodiment of the present application, the device further includes circuitry of a display module 6.

The circuitry of the display module 6 is configured to control a display screen to display the target region and the target detection region.

The method for determining the target detection region of the ionization chamber of the present application is applied to the system 10 for determining the target detection region of the ionization chamber shown in FIG. 2. The terminal 14 in the system 10 may include a mobile device 141, a tablet computer 142, a laptop computer 143, etc. The processor 12 may send a control instruction to the terminal 14, so that the target region and the target detection region are displayed on the display screen of the terminal 14. In this embodiment, the target region and the target detection region are displayed on the display screen, thus making it convenient for the operator to visually get the spatial position relationship between the target region and the target detection region, and further providing convenience for determining the target detection region of the ionization chamber.

In an embodiment, the position adjustment unit 51 is configured to move the target detection region through the display screen to make the first position of the ionization chamber coincide with the target position.

The target detection region may be moved on the display screen by inputting coordinates of the target position, dragging the mouse, and touch control, etc.

The size of the target detection region may be determined on the display screen by inputting the size of the target detection region, dragging the boundary of the region with the mouse, adjusting the boundary of the region through touch control, etc., so as to cover a relatively small part of the to-be-scanned object in photographing and achieve a better effect of the AEC.

The shape of the target detection region may also be determined on the display. A default shape of the ionization chamber is rectangular or circular, and the shape may be determined based on the default shape.

In this embodiment, the target detection region of the ionization chamber may be determined through various operations on the display screen to achieve a better imaging effect, thus further improving the determination efficiency, improving the determination accuracy, achieving a better positioning and photographing effect, ensuring the image quality, and improving the user's experience.

In an embodiment of the present application, the determination device further includes: an output module 7, a selection module 8, and a target detection region redetermining module 9.

The output module is configured to output the human body feature points and the target detection region.

The selection module is configured to take a human body feature point selected by a user as a target feature point.

The target detection region redetermining module is configured to recalculate the target region according to the position of the target feature point and readjust the target detection region, and output the target feature point and the determined target detection region.

The human body feature points and the target detection region may be outputted through the display screen in the form of text data and images. The selection of the target feature points is mainly achieved by the user selecting through the display screen, and the system activates the selection of the target feature points according to the user's selection and make the selection take effect.

Referring to FIGS. 6 and 7, when the image collector intelligently identifies a human-body part, the positions P1 and P2 of human body feature points (such as joint points) in the photographed human-body part and the target detection regions A1 and A2 of the ionization chamber may be intelligently highlighted on the human body model, and the non-selected areas are grayed. When the user expects to change the position of the automatic exposure region, a human body feature point in the target part may be activated as a target feature point, and the selection is taken into effect. The system re-executes step S12 (including steps S121 and S122) to step S15 according to the feature point expected and selected by the user, namely, the target feature point. That is, the coordinates of the position of the target feature point are re-projected, and the target region is obtained based on the target feature point, and a new position (a new first position) of the ionization chamber is calculated, and the selectable detection region of the ionization chamber is obtained according to the new first position. The target detection region of the ionization chamber is determined according to the target region and the selectable detection region so that the target detection region of the ionization chamber is within the target region, thereby realizing a manual selection and determination of the target detection region of the ionization chamber on the interface based on the human body feature points.

In an embodiment of the application, the device further includes a projection module 10.

The projection module 10 is configured to project a center identification of the target detection region and/or a boundary identification of the target detection region onto the surface of the to-be-scanned object by a projection device.

In this embodiment, for example, before scanning, the processor 12 may send the corresponding identifications of the target detection region of the ionization chamber to the projection device 16, and control the projection device 16 to project the corresponding identifications of the target detection region on the surface of the to-be-scanned object, so that the operator may visually get the spatial position relationship between the target region and the target detection region, thus further making it convenient to determine the target detection region of the ionization chamber.

FIG. 15 is a view showing a structure of an electronic device according to an embodiment of the present disclosure. The electronic device includes a memory, a processor, and a computer program stored in the memory and executable on the processor, and the processor, when executing the program, implements the method for determining the target detection region of the ionization chamber of any one of the above embodiments. The electronic device 30 shown in FIG. 15 is only an example and should not limit the function and the scope of use of the embodiments of the present disclosure.

The electronic device 30 may be in the form of a general-purpose computing device, for example, it may be a server device. The components of the electronic device 30 may include, but are not limited to: at least one processor 31, at least one memory 32, and a bus 33 connecting different system components (including the memory 32 and the processor 31.

The bus 33 includes a data bus, an address bus, and a control bus.

The memory 32 may include a volatile memory, such as a random access memory (RAM) 321 and/or a cache memory 322, and may further include a read-only memory (ROM) 323.

The memory 32 may also include a group of (at least one) program modules 324 program/application tools 325, and such program modules 324 include but are not limited to: an operating system, one or more application programs, other program modules, and program data, and each of or some combination of these examples may include an implementation of a network environment.

The processor 31, when running the computer program stored in the memory 32, executes various functional applications and data processing, such as the method for determining the target detection region of the ionization chamber in any one of the above embodiments of the disclosure.

The electronic device 30 may also communicate with one or more external devices 34 (e.g., buttons, pointing devices, etc.). Such a communication may be performed via an input/output (I/O) interface 35. Furthermore, the electronic device 30 for generating a model may also communicate with one or more networks (e.g., a local area network, a wide area network, and/or a public network, such as the Internet) via a network adapter 36. As shown in the figure, the network adapter 36 communicates with other modules of the electronic device 30 generating the model via a bus 33. It should be understood that, although not shown in the figure, other hardware and/or software modules, including but are not limited to microcode, a device driver, a redundant processor, an external disk drive array, a Redundant Access Independent Disk system, a tape driver, and a data backup storage system, etc., may be used in conjunction with the electronic device 30 generating the model.

It should be noted that various modules/circuitries of modules, or submodules/submodules of the electronic device are mentioned in the above detailed description, such divisions are merely exemplary and not mandatory. In fact, according to the embodiments of the disclosure, the features and functions of two or more modules/ circuitries of modules described above can be embodied in one module/ a module. Conversely, the features and functions of one module/ one a module described above may be further embodied by dividing into multiple modules.

This embodiment provides a non-transitory computer-readable storage medium on which a computer program is stored. The program, when being executed by a processor, performs any one of the methods for determining the target detection region of the ionization chamber above.

The readable storage medium may include but is not limited to: a portable disk, a hard disk, a random-access memory, a read-only memory, an erasable programmable read-only memory, an optical storage device, a magnetic storage device, or any suitable combination thereof.

In a possible implementation, the present disclosure may also be implemented in the form of a program product, which includes program codes. When the program product is run on a terminal device, the program codes cause the terminal device to execute any one of the methods for determining the target detection region of the ionization chamber in the above embodiments.

The program codes of the present disclosure may be written and executed in one of or any one combination of one or more programming languages, and the program codes may be executed completely or partially on the user device, may be executed as an independent software package, may be executed partially on the user device and partially on a remote device, or may be executed completely on the remote device.

## Claims

1. A method for determining a target detection region of an ionization chamber, the ionization chamber comprising a moving unit with a driving device and a selectable detection region including a plurality of dose receiving units, the method comprising:
obtaining an image comprising a target region of a to-be-scanned object acquired by an image collector;
identifying the target region based on the image;
obtaining a first position of the ionization chamber; and
obtaining a selectable detection region of the ionization chamber based on the first position;
the method further comprising:
when the target region is outside the selectable detection region, obtaining a target position of the ionization chamber, and controlling the driving device of the ionization chamber to move the ionization chamber to enable the target position to coincide with the first position; wherein when the ionization chamber is located at the target position, the selectable detection region of the ionization chamber at least partially overlaps the target region;
when the target region is within the selectable detection region, selecting and determining the target detection region from the selectable detection region, wherein a minimum distance between a boundary of the target detection region and a boundary of the target region is within a preset boundary distance, and the target detection region of the ionization chamber is within the target region.

2. The method for determining the target detection region of the ionization chamber according to claim 1, wherein identifying the target region based on the image comprises:
identifying a plurality of human body feature points based on the image, and obtaining second positions of the plurality of human body feature points in the spatial coordinate system; and
determining the target region according to the plurality of human body feature points and the second positions.

3. The method for determining the target detection region of the ionization chamber according to claim 2, wherein determining the target region according to the plurality of human body feature points and the second positions includes:
determining target human body feature points corresponding to a target human-body part according to the plurality of human body feature points;
taking a center of the second positions corresponding to the target human feature points as a center point, and determining a region within a preset distance from the center point as the target region.

4. The method for determining the target detection region of the ionization chamber according to any one of claims 1-3, further comprising: controlling a display screen to display the target region, the selectable detection region, or the target detection region.

5. The method for determining the target detection region of the ionization chamber according to claim 1, further comprising: controlling a display screen to display the target region and the target detection region;
wherein the target detection region is moved on the display screen to make the first position of the ionization chamber coincide with the target position.

6. The method for determining the target detection region of the ionization chamber according to claim 2, further comprising:
outputting the plurality of human body feature points and the target detection region;
taking a human body feature point selected by a user as a target feature point; and
recalculating the target region according to the position of the target feature point and redetermining the target detection region, and outputting the target feature point and the redetermined target detection region.

7. The method for determining the target detection region of the ionization chamber according to any one of claims 1-6, further comprising: controlling a projection device to project at least one of a center identification of the target detection region and a boundary identification of the target detection region onto a surface of the to-be-scanned object.

8. The method for determining the target detection region of the ionization chamber according to any one of claims 1-7, wherein:
the ionization chamber comprises one or more dose receiving units;
the selectable detection region of the ionization chamber comprises all of the one or more dose receiving units of the ionization chamber; and
the target detection region of the ionization chamber comprises only dose receiving units in a working state.

9. The method for determining the target detection region of the ionization chamber according to claim 8, wherein the ionization chamber is a dose-receiving-unit array consisting of multiple dose receiving units.

10. The method for determining the target detection region of the ionization chamber according to claim 2, wherein, identifying the plurality of human body feature points based on the image, and obtaining the second positions of the plurality of human body feature points in the spatial coordinate system, comprise:
identifying the plurality of human body feature points in an image;
constructing a human body model in the spatial coordinate system; and
determining positions of the plurality of human body feature points in a three-dimensional spatial coordinate system based on positions of the plurality of human body feature points in the human body model, wherein the positions of the plurality of human body feature points in the three-dimensional spatial coordinate system are the second positions.

11. The method for determining the target detection region of the ionization chamber according to claim 10, wherein identifying the plurality of human body feature points in the image, comprises:
inputting sample images marked with human body feature points into a neural network model to train a recognition model for the human body feature points;
inputting the image comprising the target region of the to-be-scanned object acquired by the image collector into the trained recognition model to identify the plurality of human body feature points.

12. A device for determining a target detection region of an ionization chamber, the ionization chamber comprising a moving unit with a driving device and a selectable detection region including a plurality of dose receiving units, the device comprising:
an image acquiring module (1) configured to obtain an image comprising a target region of a to-be-scanned object acquired by an image collector;
an image recognizing module (2) configured to identify the target region based on the image;
a first position obtaining module (3) configured to obtain a first position of the ionization chamber in a spatial coordinate system; and
a selectable detection region calculating module (4) configured to obtain a selectable detection region of the ionization chamber based on the first position;
the device further comprising a target detection region determining module (5) configured to, when the target region is outside the selectable detection region, obtain a target position of the ionization chamber, and control the driving device of the ionization chamber to move the ionization chamber to enable the target position to coincide with the first position, wherein when the ionization chamber is located at the target position, the selectable detection region of the ionization chamber at least partially overlaps the target region; and when the target region is within the selectable detection region, select and determine the target detection region from the selectable detection region, wherein a minimum distance between a boundary of the target detection region and a boundary of the target region is within a preset boundary distance, and the target detection region of the ionization chamber is within the target region.

13. An electronic device (30) comprising a memory (32) and a processor (31), wherein the memory (32) stores a computer program executable on the processor (31), wherein the processor (31), when executing the computer program, performs the method for determining the target detection region of the ionization chamber according to any one of claims 1 to 11.

14. A non-transitory computer-readable storage medium, having a computer program stored thereon, wherein the computer program, when being executed by a processor, performs the method for determining the target detection region of the ionization chamber according to any one of claims 1 to 11.

## Patentansprüche

1. Ein Verfahren zum Bestimmen eines Zieldetektionsbereichs einer Ionisationskammer, wobei die Ionisationskammer eine Bewegungseinheit mit einer Antriebsvorrichtung und einen auswählbaren Detektionsbereich, der eine Vielzahl von Dosisaufnahmeeinheiten umfasst, beinhaltet, wobei das Verfahren Folgendes beinhaltet:
Erhalten eines Bildes, das einen Zielbereich eines abzutastenden Objekts beinhaltet, das von einem Bildsammler erfasst wird;
Identifizieren des Zielbereichs basierend auf dem Bild;
Erhalten einer ersten Position der Ionisationskammer; und
Erhalten eines auswählbaren Detektionsbereichs der Ionisationskammer basierend auf der ersten Position;
wobei das Verfahren ferner Folgendes beinhaltet:
wenn sich der Zielbereich außerhalb des auswählbaren Detektionsbereichs befindet, Erhalten einer Zielposition der Ionisationskammer und Steuern der Antriebsvorrichtung der Ionisationskammer, um die Ionisationskammer zu bewegen, um zu ermöglichen, dass die Zielposition mit der ersten Position zusammenfällt; wobei, wenn sich die Ionisationskammer an der Zielposition befindet, der auswählbare Detektionsbereich der Ionisationskammer den Zielbereich mindestens teilweise überlappt;
wenn sich der Zielbereich innerhalb des auswählbaren Detektionsbereichs befindet, Auswählen und Bestimmen des Zieldetektionsbereichs aus dem auswählbaren Detektionsbereich, wobei ein Mindestabstand zwischen einer Grenze des Zieldetektionsbereichs und einer Grenze des Zielbereichs innerhalb eines voreingestellten Grenzabstands liegt und der Zieldetektionsbereich der Ionisationskammer innerhalb des Zielbereichs liegt.

2. Verfahren zum Bestimmen des Zieldetektionsbereichs der Ionisationskammer gemäß Anspruch 1, wobei das Identifizieren des Zielbereichs basierend auf dem Bild Folgendes beinhaltet:
Identifizieren einer Vielzahl von Merkmalspunkten des menschlichen Körpers basierend auf dem Bild und Erhalten von zweiten Positionen der Vielzahl von Merkmalspunkten des menschlichen Körpers in dem räumlichen Koordinatensystem; und
Bestimmen des Zielbereichs gemäß der Vielzahl von Merkmalspunkten des menschlichen Körpers und den zweiten Positionen.

3. Verfahren zum Bestimmen des Zieldetektionsbereichs der Ionisationskammer gemäß Anspruch 2, wobei das Bestimmen des Zielbereichs gemäß der Vielzahl von Merkmalspunkten des menschlichen Körpers und den zweiten Positionen Folgendes umfasst:
Bestimmen von Zielmerkmalspunkten des menschlichen Körpers, die einem Zielteil des menschlichen Körpers entsprechen, gemäß der Vielzahl von Merkmalspunkten des menschlichen Körpers;
Nehmen einer Mitte der zweiten Positionen, die den Zielmerkmalspunkten des menschlichen Körpers entsprechen, als einen Mittelpunkt und Bestimmen eines Bereichs innerhalb eines voreingestellten Abstands von dem Mittelpunkt als den Zielbereich.

4. Verfahren zum Bestimmen des Zieldetektionsbereichs der Ionisationskammer gemäß einem der Ansprüche 1-3, das ferner Folgendes beinhaltet: Steuern eines Anzeigebildschirms, um den Zielbereich, den auswählbaren Detektionsbereich oder den Zieldetektionsbereich anzuzeigen.

5. Verfahren zum Bestimmen des Zieldetektionsbereichs der Ionisationskammer gemäß Anspruch 1, das ferner Folgendes beinhaltet: Steuern eines Anzeigebildschirms, um den Zielbereich und den Zieldetektionsbereich anzuzeigen;
wobei der Zieldetektionsbereich auf dem Anzeigebildschirm bewegt wird, um zu bewirken, dass die erste Position der Ionisationskammer mit der Zielposition zusammenfällt.

6. Verfahren zum Bestimmen des Zieldetektionsbereichs der Ionisationskammer gemäß Anspruch 2, das ferner Folgendes beinhaltet:
Ausgeben der Vielzahl von Merkmalspunkten des menschlichen Körpers und des Zieldetektionsbereichs;
Nehmen eines Merkmalspunkts des menschlichen Körpers, der von einem Benutzer ausgewählt wurde, als einen Zielmerkmalspunkt; und
Neuberechnen des Zielbereichs gemäß der Position des Zielmerkmalspunkts und Neubestimmen des Zieldetektionsbereichs und Ausgeben des Zielmerkmalspunkts und des neubestimmten Zieldetektionsbereichs.

7. Verfahren zum Bestimmen des Zieldetektionsbereichs der Ionisationskammer gemäß einem der Ansprüche 1-6, das ferner Folgendes beinhaltet: Steuern einer Projektionsvorrichtung, um mindestens eines von einer Mittenidentifikation des Zieldetektionsbereichs und einer Grenzenidentifikation des Zieldetektionsbereichs auf eine Oberfläche des abzutastenden Objekts zu projizieren.

8. Verfahren zum Bestimmen des Zieldetektionsbereichs der Ionisationskammer gemäß einem der Ansprüche 1-7, wobei:
die Ionisationskammer eine oder mehrere Dosisaufnahmeeinheiten beinhaltet;
der auswählbare Detektionsbereich der Ionisationskammer alle der einen oder mehreren Dosisaufnahmeeinheiten der Ionisationskammer beinhaltet; und
der Zieldetektionsbereich der Ionisationskammer nur Dosisaufnahmeeinheiten in einem Arbeitszustand beinhaltet.

9. Verfahren zum Bestimmen des Zieldetektionsbereichs der Ionisationskammer gemäß Anspruch 8, wobei die Ionisationskammer ein Dosisaufnahmeeinheitsarray ist, das aus mehreren Dosisaufnahmeeinheiten besteht.

10. Verfahren zum Bestimmen des Zieldetektionsbereichs der Ionisationskammer gemäß Anspruch 2, wobei das Identifizieren der Vielzahl von Merkmalspunkten des menschlichen Körpers basierend auf dem Bild und das Erhalten der zweiten Positionen der Vielzahl von Merkmalspunkten des menschlichen Körpers in dem räumlichen Koordinatensystem Folgendes beinhalten:
Identifizieren der Vielzahl von Merkmalspunkten des menschlichen Körpers in einem Bild;
Konstruieren eines Modells des menschlichen Körpers in dem räumlichen Koordinatensystem; und
Bestimmen von Positionen der Vielzahl von Merkmalspunkten des menschlichen Körpers in einem dreidimensionalen räumlichen Koordinatensystem basierend auf Positionen der Vielzahl von Merkmalspunkten des menschlichen Körpers in dem Modell des menschlichen Körpers, wobei die Positionen der Vielzahl von Merkmalspunkten des menschlichen Körpers in dem dreidimensionalen räumlichen Koordinatensystem die zweiten Positionen sind.

11. Verfahren zum Bestimmen des Zieldetektionsbereichs der Ionisationskammer gemäß Anspruch 10, wobei das Identifizieren der Vielzahl von Merkmalspunkten des menschlichen Körpers in dem Bild Folgendes beinhaltet:
Eingeben von Musterbildern, die mit Merkmalspunkten des menschlichen Körpers markiert sind, in ein neuronales Netzwerkmodell, um ein Erkennungsmodell für die Merkmalspunkte des menschlichen Körpers zu trainieren;
Eingeben des Bildes, das den Zielbereich des abzutastenden Objekts beinhaltet, das von dem Bildsammler erfasst wird, in das trainierte Erkennungsmodell, um die Vielzahl von Merkmalspunkten des menschlichen Körpers zu identifizieren.

12. Eine Vorrichtung zum Bestimmen eines Zieldetektionsbereichs einer Ionisationskammer, wobei die Ionisationskammer eine Bewegungseinheit mit einer Antriebsvorrichtung und einen auswählbaren Detektionsbereich beinhaltet, der eine Vielzahl von Dosisaufnahmeeinheiten umfasst, wobei die Vorrichtung Folgendes beinhaltet:
ein Bilderfassungsmodul (1), das konfiguriert ist, um ein Bild zu erhalten, das einen Zielbereich eines abzutastenden Objekts beinhaltet, das von einem Bildsammler erfasst wird;
ein Bilderkennungsmodul (2), das konfiguriert ist, um den Zielbereich basierend auf dem Bild zu identifizieren;
ein erstes Positionserhaltungsmodul (3), das konfiguriert ist, um eine erste Position der Ionisationskammer in einem räumlichen Koordinatensystem zu erhalten; und
ein auswählbares Detektionsbereichsberechnungsmodul (4), das konfiguriert ist, um einen auswählbaren Detektionsbereich der Ionisationskammer basierend auf der ersten Position zu erhalten;
wobei die Vorrichtung ferner ein Zieldetektionsbereichsbestimmungsmodul (5) beinhaltet, das konfiguriert ist, um, wenn sich der Zielbereich außerhalb des auswählbaren Detektionsbereichs befindet, eine Zielposition der Ionisationskammer zu erhalten und die Antriebsvorrichtung der Ionisationskammer zu steuern, um die Ionisationskammer zu bewegen, um zu ermöglichen, dass die Zielposition mit der ersten Position zusammenfällt, wobei, wenn sich die Ionisationskammer an der Zielposition befindet, der auswählbare Detektionsbereich der Ionisationskammer den Zielbereich mindestens teilweise überlappt; und wenn sich der Zielbereich innerhalb des auswählbaren Detektionsbereichs befindet, Auswählen und Bestimmen des Zieldetektionsbereichs aus dem auswählbaren Detektionsbereich, wobei ein Mindestabstand zwischen einer Grenze des Zieldetektionsbereichs und einer Grenze des Zielbereichs innerhalb eines voreingestellten Grenzabstands liegt und der Zieldetektionsbereich der Ionisationskammer innerhalb des Zielbereichs liegt.

13. Eine elektronische Vorrichtung (30), die einen Speicher (32) und einen Prozessor (31) beinhaltet, wobei der Speicher (32) ein Computerprogramm speichert, das auf dem Prozessor (31) ausführbar ist, wobei der Prozessor (31), wenn er das Computerprogramm ausführt, das Verfahren zum Bestimmen des Zieldetektionsbereichs der Ionisationskammer gemäß einem der Ansprüche 1 bis 11 durchführt.

14. Ein nicht transitorisches computerlesbares Speichermedium, auf dem ein Computerprogramm gespeichert ist, wobei das Computerprogramm, wenn es von einem Prozessor ausgeführt wird, das Verfahren zum Bestimmen des Zieldetektionsbereichs der Ionisationskammer gemäß einem der Ansprüche 1 bis 11 durchführt.

## Revendications

1. Un procédé pour la détermination d'une région de détection cible d'une chambre d'ionisation, la chambre d'ionisation comprenant une unité mobile avec un dispositif d'entraînement et une région de détection sélectionnable incluant une pluralité d'unités de réception de dose, le procédé comprenant
l'obtention d'une image comprenant une région cible d'un objet à scanner acquise par un collecteur d'image ;
l'identification de la région cible sur la base de l'image ;
l'obtention d'une première position de la chambre d'ionisation ; et
l'obtention d'une région de détection sélectionnable de la chambre d'ionisation sur la base de la première position ;
le procédé comprenant en outre :
lorsque la région cible est à l'extérieur de la région de détection sélectionnable, l'obtention d'une position cible de la chambre d'ionisation, et la commande du dispositif d'entraînement de la chambre d'ionisation pour déplacer la chambre d'ionisation afin de permettre à la position cible de coïncider avec la première position ; dans lequel lorsque la chambre d'ionisation est située à la position cible, la région de détection sélectionnable de la chambre d'ionisation chevauche au moins partiellement la région cible ;
lorsque la région cible est à l'intérieur de la région de détection sélectionnable, la sélection et la détermination de la région de détection cible à partir de la région de détection sélectionnable, dans lequel une distance minimale entre une limite de la région de détection cible et une limite de la région cible est à l'intérieur d'une distance limite prédéfinie, et la région de détection cible de la chambre d'ionisation est à l'intérieur de la région cible.

2. Le procédé pour la détermination de la région de détection cible de la chambre d'ionisation selon la revendication 1, dans lequel l'identification de la région cible sur la base de l'image comprend :
l'identification d'une pluralité de points caractéristiques de corps humain sur la base de l'image, et l'obtention de deuxièmes positions de la pluralité de points caractéristiques de corps humain dans le système de coordonnées spatiales ; et
la détermination de la région cible selon la pluralité de points caractéristiques de corps humain et les deuxièmes positions.

3. Le procédé pour la détermination de la région de détection cible de la chambre d'ionisation selon la revendication 2, dans lequel la détermination de la région cible selon la pluralité de points caractéristiques de corps humain et les deuxièmes positions inclut :
la détermination de points caractéristiques de corps humain cibles correspondant à une partie de corps humain cible selon la pluralité de points caractéristiques de corps humain ;
la prise d'un centre des deuxièmes positions correspondant aux points caractéristiques humains cibles en tant que point central, et la détermination d'une région à l'intérieur d'une distance prédéfinie à partir du point central en tant que région cible.

4. Le procédé pour la détermination de la région de détection cible de la chambre d'ionisation selon l'une quelconque des revendications 1 à 3, comprenant en outre : la commande d'un écran d'affichage pour afficher la région cible, la région de détection sélectionnable, ou la région de détection cible.

5. Le procédé pour la détermination de la région de détection cible de la chambre d'ionisation selon la revendication 1, comprenant en outre : la commande d'un écran d'affichage pour afficher la région cible et la région de détection cible ;
dans lequel la région de détection cible est déplacée sur l'écran d'affichage pour faire coïncider la première position de la chambre d'ionisation avec la position cible.

6. Le procédé pour la détermination de la région de détection cible de la chambre d'ionisation selon la revendication 2, comprenant en outre :
la sortie de la pluralité de points caractéristiques de corps humain et de la région de détection cible ;
la prise d'un point caractéristique de corps humain sélectionné par un utilisateur en tant que point caractéristique cible ; et
le recalcul de la région cible selon la position du point caractéristique cible et la redétermination de la région de détection cible, et la sortie du point caractéristique cible et de la région de détection cible redéterminée.

7. Le procédé pour la détermination de la région de détection cible de la chambre d'ionisation selon l'une quelconque des revendications 1 à 6, comprenant en outre : la commande d'un dispositif de projection pour projeter au moins une identification parmi une identification de centre de la région de détection cible et une identification de limite de la région de détection cible sur une surface de l'objet à scanner.

8. Le procédé pour la détermination de la région de détection cible de la chambre d'ionisation selon l'une quelconque des revendications 1 à 7, dans lequel :
la chambre d'ionisation comprend une ou plusieurs unités de réception de dose ;
la région de détection sélectionnable de la chambre d'ionisation comprend la totalité des une ou plusieurs unités de réception de dose de la chambre d'ionisation ; et
la région de détection cible de la chambre d'ionisation comprend uniquement des unités de réception de dose dans un état de travail.

9. Le procédé pour la détermination de la région de détection cible de la chambre d'ionisation selon la revendication 8, dans lequel la chambre d'ionisation est un réseau d'unités de réception de dose constitué de multiples unités de réception de dose.

10. Le procédé pour la détermination de la région de détection cible de la chambre d'ionisation selon la revendication 2, dans lequel, l'identification de la pluralité de points caractéristiques de corps humain sur la base de l'image, et l'obtention des deuxièmes positions de la pluralité de points caractéristiques de corps humain dans le système de coordonnées spatiales, comprennent :
l'identification de la pluralité de points caractéristiques de corps humain dans une image ;
la construction d'un modèle de corps humain dans le système de coordonnées spatiales ; et
la détermination de positions de la pluralité de points caractéristiques de corps humain dans un système de coordonnées spatiales tridimensionnel sur la base de positions de la pluralité de points caractéristiques de corps humain dans le modèle de corps humain, dans lequel les positions de la pluralité de points caractéristiques de corps humain dans le système de coordonnées spatiales tridimensionnel sont les deuxièmes positions.

11. Le procédé pour la détermination de la région de détection cible de la chambre d'ionisation selon la revendication 10, dans lequel l'identification de la pluralité de points caractéristiques de corps humain dans l'image comprend :
l'entrée d'images échantillons marquées avec des points caractéristiques de corps humain dans un modèle de réseau neuronal pour former un modèle de reconnaissance pour les points caractéristiques de corps humain ;
l'entrée de l'image comprenant la région cible de l'objet à scanner acquise par le collecteur d'image dans le modèle de reconnaissance formé pour identifier la pluralité de points caractéristiques de corps humain.

12. Un dispositif pour la détermination d'une région de détection cible d'une chambre d'ionisation, la chambre d'ionisation comprenant une unité mobile avec un dispositif d'entraînement et une région de détection sélectionnable incluant une pluralité d'unités de réception de dose, le dispositif comprenant
un module d'acquisition d'image (1) configuré pour obtenir une image comprenant une région cible d'un objet à scanner acquise par un collecteur d'image ;
un module de reconnaissance d'image (2) configuré pour identifier la région cible sur la base de l'image ;
un module d'obtention de première position (3) configuré pour obtenir une première position de la chambre d'ionisation dans un système de coordonnées spatiales ; et
un module de calcul de région de détection sélectionnable (4) configuré pour obtenir une région de détection sélectionnable de la chambre d'ionisation sur la base de la première position ;
le dispositif comprenant en outre un module de détermination de région de détection cible (5) configuré pour, lorsque la région cible est à l'extérieur de la région de détection sélectionnable, obtenir une position cible de la chambre d'ionisation, et commander le dispositif d'entraînement de la chambre d'ionisation pour déplacer la chambre d'ionisation afin de permettre à la position cible de coïncider avec la première position, dans lequel lorsque la chambre d'ionisation est située à la position cible, la région de détection sélectionnable de la chambre d'ionisation chevauche au moins partiellement la région cible ; et lorsque la région cible est à l'intérieur de la région de détection sélectionnable, sélectionner et déterminer la région de détection cible à partir de la région de détection sélectionnable, dans lequel une distance minimale entre une limite de la région de détection cible et une limite de la région cible est à l'intérieur d'une distance limite prédéfinie, et la région de détection cible de la chambre d'ionisation est à l'intérieur de la région cible.

13. Un dispositif électronique (30) comprenant une mémoire (32) et un processeur (31), dans lequel la mémoire (32) stocke un programme informatique exécutable sur le processeur (31), dans lequel le processeur (31), lors de l'exécution du programme informatique, effectue le procédé pour la détermination de la région de détection cible de la chambre d'ionisation selon l'une quelconque des revendications 1 à 11.

14. Un support de stockage non transitoire lisible par ordinateur, sur lequel est stocké un programme informatique, dans lequel le programme informatique, lorsqu'il est exécuté par un processeur, effectue le procédé pour la détermination de la région de détection cible de la chambre d'ionisation selon l'une quelconque des revendications 1 à 11.
